(19) Europäisches Patentamt
European Patent Office
Office européen des brevets

(11) Publication number: **0 043 141**
**B1**

(12) # EUROPEAN PATENT SPECIFICATION

(45) Date of publication of patent specification: **12.09.84**

(21) Application number: **81105115.0**

(22) Date of filing: **01.07.81**

(51) Int. Cl.³: **C 07 D 295/10,** C 07 C 97/03, C 07 D 317/58 // C07C49/235, C07C149/32, C07C147/12, C07C121/78

(54) **Pharmaceutical compositions having antiviral activity for topical administration and the use of 1-(substituted phenyl)-5-(4-morpholinyl)-1-penten-3-one compounds for the preparation of pharmaceutical compositions having an antiviral effect.**

(30) Priority: **02.07.80 US 165430**

(43) Date of publication of application:
**06.01.82 Bulletin 82/01**

(45) Publication of the grant of the patent:
**12.09.84 Bulletin 84/37**

(84) Designated Contracting States:
**AT BE CH DE FR GB IT LI NL SE**

(56) References cited:
**US-A-4 173 649**
**JOURNAL OF PHARMACEUTICAL SCIENCES, vol. 65, no. 4, April 1976, WASHINGTON D.C. (US), J.R. DIMMOCK et al.: "Evaluation of Mannich Bases and Related Compounds as Inhibitors of Mitochondrial Function in Yeast and Inhibition of Blood Platelet Aggregation, Blood Clotting and In Vitro Metabolism of 5-Dimethyl-1-amino-1-phenyl-1-penten-3-one Hydrochloride", pages 482-488**

**The file contains technical information submitted after the application was filed and not included in this specification**

(73) Proprietor: **MERRELL DOW PHARMACEUTICALS INC.**
**2110 E. Galbraith Road**
**Cincinnati Ohio 45215 (US)**

(72) Inventor: **Ritter, Harry W.**
**324 Miami Valley Drive**
**Loveland Ohio 45140 (US)**
Inventor: **Edwards, Michael L.**
**12033 Deerhorn Drive**
**Cincinnati Ohio 45240 (US)**

(74) Representative: **Vossius Vossius Tauchner Heunemann Rauh**
**Siebertstrasse 4 P.O. Box 86 07 67**
**D-8000 München 86 (DE)**

(56) References cited:
**JOURNAL OF PHARMACEUTICAL SCIENCES, vol. 54, 1965, WASHINGTON D.C. (US), C.H. BRUENING et al.: "Synthetic Relatives of Reserpine", pages 925,926**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 043 141 B1

(56) References cited:

CHEMICAL ABSTRACTS, vol. 64, no. 10, 9th May, 1966, column 14790e-f, COLUMBUS, OHIO (US), YA. I. ZAIDLER et al.: "Antiarrhythmic activity of beta-amino ketones"

CHEMICAL ABSTRACTS, vol. 50, no. 12, 25th June, 1956, column 8888a-d, COLUMBUS, OHIO (US), J. PORSZASZ et al.: "Pharmacology of amino ketones with nicotinic and antinicotinic effects II"

J. CHEM. SOC., 1938, ref. 234, LONDON (GB), H.B. NISBET: "Pyrazoline Local Anaesthetics. Part I. Derivatives of Benzylidene- and Anisylidene-acetone", pages 1237-1241

J. CHEM. SOC. 1938, ref. 293, LONDON (GB), H.B. NISBET: "Pyrazoline Local Anaesthetics. Part II. Derivatives of Alkylated 3:4-Dihydroxybenzyl-ideneacetones", pages 1568-1571

J. CHEM. SOC., 1938, ref. 294, LONDON (GB), G.A. LEVVY et al.: "Pyrazoline Local Anaesthetics. Part III. Derivatives of o-Alkoxybenzylidene-acetones", pages 1572-1574

CHEMICAL ABSTRACTS, vol. 22, no. 6, 20th March, 1928, pages 963-964, COLUMBUS, OHIO (US), C. MANNICH et al.: "Synthesis of unsaturated ketones and their reduction products"

CHEMICAL ABSTRACTS, vol. 51, no. 13, 10th July, 1957, column 9909d-e, COLUMBUS, OHIO (US), J. PORSZASZ et al.: "Pharmacology of aminoketones. III. Comparison of Largactil, Pacatal, and F-933 (2-(piperidinomethyl)-1,4-benzodioxan) and the Sixth Collective Index, Subjects Mi-Pk, page 8514s, 2nd column

J. AM. CHEM. SOC., vol. 73, Oct. 1951, WASHINGTON D.C. (US), J.H. BURCKHALTER et al.: "Mannich Bases from Benzalacetones", pages 4835-4836

CHEMICAL ABSTRACTS, vol. 47, no. 12, 25th June, 1953, column 5932i-5932d, COLUMBUS, OHIO (US), L.S. EFROS et al.: "Benzothiazole derivatives. Preparation of 1-benzothiazolyl-3-methyl-5(4H)-pyrazolone"

CHEMICAL ABSTRACTS, vol. 53, no. 5, 10th March, 1959, column 4185a-4186e, COLUMBUS, Ohio (US), R. ANDRISANO et al.: "Action of hydroxylamine on Mannich bases of benzylidenacetone"

CHEMICAL ABSTRACTS, vol. 52, no. 13, 10th July, 1958, column 11067b-i, COLUMBUS, OHIO (US), L.W. NOBLES et al.: "Ketonic Mannich bases and the products of their reduction and bromination"

J. AM. PHARMACEUTICAL ASSOCIATION, vol. 49, no. 5, May 1960, WASHINGTON D.C. (US), E.D. TAYLOR et al.: "Some Ketonic Mannich Bases", pages 317-319

(56) References cited:

ANNALES PHARMACEUTIQUES FRANCAISES, vol. 32, no. 12, 1974, PARIS (FR) A. PSARREA-SANDRIS: "Synthèse de dérivés de l'acide férulique. III. Amides et bases de Mannich de la vanillalacétone" pages 685-696

CHEMICAL ABSTRACTS, vol. 76, no. 17, 24th April, 1972, page 450, abstract 99583w, COLUMBUS, OHIO (US), A. GVOZDJAKOVA et al.: "Aminomethylation of alpha.beta-unsaturated ketones"

CHEMICAL ABSTRACTS, vol. 66, no. 9, 27th February, 1967, page 3564, abstract 37578h, COLUMBUS, OHIO (US), E.G. DARBINYAN et al.: "Synthesis and polymerization of beta-aryl substituted divinyl ketones"

CAN. J. CHEM., vol. 58, no. 10, May 1980, OTTAWA (CA), J.R. DIMMOCK et al.: "The reaction of some nuclear substituted acyclic conjugated styryl ketones and related Mannich bases with ethanethiol", pages 984-991

**Description**

Many 1-aryl-5-amino-1-penten-3-ones are known. Several pharmacological activities are reported for such compounds. However, there has been no suggestion that such compounds, either known or heretofore unknown, might possess antiviral activity.

Summary of the Invention

Accordingly, it is an object of this invention to provide pharmaceutical compositions for treating a viral infection which is amenable to topical treatment containing an anti-virally effective amount, e.g. an amount effective for treating herpes virus topically, of a compound of formula I

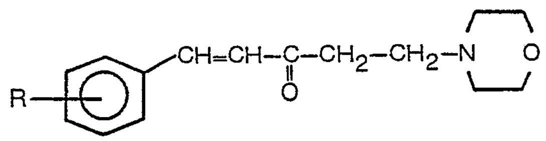
(I)

wherein R is either (a) a mono substituted moiety selected from methoxy, 4-halo, 3-halo, $CF_3$, $—S—C_{1-4}$-alkyl, $—SO_2C_{1-4}$-alkyl, or (b) a dihalo moiety; or a pharmaceutically acceptable salt thereof with an acid or a quaternary ammonium salt thereof.

Discussion of the Prior Art

J. Pharm. Sciences, Vol. 65, No. 4, April 1976, 482—488: This publication relates only to 1-phenyl-1-penten-3-ones having a 5-dialkylamino moiety.

J. Pharm. Sciences, Vol. 54, 1965, 925—926:
This publication relates to a narrow group of compounds, e.g. 1-(trimethoxyphenyl)-1-penten-3-ones having a 5-diethylamino moiety. These compounds are used as CNS depressants. This application excludes the tri-methoxysubstitution.

Chem. Abs., Vol 64, No. 10, 9th May 1966, col. 14790 e-f:
This publication relates to a 3,4-methylene-dioxy-1-pentene-3-one which is outside the scope of the present invention.

Chem. Abs., Vol. 50, No. 12, 25th June 1956, col. 8888 a-d:
This publication relates inter alia to 1-phenyl-5-(pyrrolidino or piperidino)-1-pentene-3-ones as anti-hypertensives. The scope of the present invention does not overlap with this publication.

J. Am. Chem. Soc., Vol. 73, October 1951, p. 4836:
This publication relates primarily to compounds having a 5-dialkylamino or a 5-morpholino moiety. These compounds do not overlap with the compounds of the present invention.

Chem. Abs., Vol. 52, No. 13, 10th July 1958, col. 11067, b-i:
This publication refers to ketonic Mannich bases and their reaction products which have no impact on the scope of this invention.

J. Am. Pharm. Ass., Vol. 49, No. 5, May 1960:
This publication refers to some ketonic Mannich bases with a piperidino group. It is outside the scope of the present invention.

Chem. Abs., Vol, 76, No. 17, 24th April 1972, 450, Abst. 99583 w:
This publication has 5-morpholino compounds having $—O—CH_2—O$, $NO_2$, dimethylamino, 2-Cl or 4-$NO_2$ substituted phenyl compounds. The scope of the present invention does not include the compounds of this publication. Besides, there is no discussion on use of these compounds as topical agents.

Detailed Discussion

In all of the compositions of this invention suitable halo atoms as R include F, Cl, Br and I.

In general, on the phenyl ring to which R is attached monosubstitution in the 3- or 4-position is preferred. If R is halo, disubstitution is also preferred, e.g. in the 3,4-positions.

As R, preferred is halo (especially Cl).

The quaternary ammonium salts of the compounds of Formula I include those which are formed with e.g., alkyl halides such as alkyl iodides, alkyl chlorides or alkyl bromides. Suitable alkyl moieties include $C_{1-4}$-alkyls.

Also included are those quaternaries prepared from the sulfonic acids such as methyl sulfonic acid, toluenesulfonic acid and the like, as well as any of the normal prior art quaternary salts generally utilized for pharmaceutical formulation benefits. The preferred quaternaries are those formed with lower (e.g. $C_{1-4}$) alkyl halides.

The hydrochloride salts, in general, are the preferred salts as well as the preferred forms of the compounds I.

Illustrative examples of the compounds I include the following:

5-(4-morpholinyl)-1-(3,4-dichlorophenyl)-1-penten-3-one;

5-(4-morpholinyl)-1-(3-trifluoromethylphenyl)-1-penten-3-one;
5-(4-morpholinyl)-1-(2,6-dichlorophenyl)-1-penten-3-one;
5-(4-morpholinyl)-1-(4-methylthiophenyl)-1-penten-3-one;
5-(4-morpholinyl)-1-(4-methylsulfonylphenyl)-1-penten-3-one;
5-(4-morpholinyl)-1-(4-chlorophenyl)-1-penten-3-one;

Illustrative examples of the compounds I also include the hydrochloride salts of each of the foregoing compounds, e.g. the compounds prepared in the examples.

The compounds I are useful as topical antiviral agents. For example, they may be used in the topical treatment of *Herpesvirus hominis,* a DNA virus, which causes disease for which, at the present time, there is no completely adequate treatment; Vaccinia, Cytomegalovirus; and other DNA viruses which are amenable to topical treatment.

The compounds I may be used in the control of all manifestations of *Herpesvirus hominis* which are amenable to topically administered agents, which excludes, e.g., herpes encephalitis and generalized infections. Herpesvirus-induced indications which may be treated using the compounds I include acute gingivostomatitis, *Herpes labialis,* outer eye infections, such as keratoconjunctivitis, *Herpes genitalia,* neonatal herpes, etc. Both *Herpesvirus hominis,* type 1 and *Herpevirus hominis,* type 2 are amenable to topical control using the compounds I. Both types are capable of infecting either the upper sites of the body or the genitalia. Additionally, chicken pox, a primary infection caused by a herpes virus (Varicella-Zoster) and its secondary manifestation, shingles (Herpes-Zoster) can also be topically treated with the compounds I.

The compounds I may be applied in various topical formulations to the infected site in the host suffering, e.g., from the effects of *Herpesvirus hominis,* e.g. a mammal or other animal, including humans and rats, cats, dogs, pigs, cattle, horses, rabbits, fowl, etc. As used herein, the term "patient" is intended to mean the animal or mammal being treated.

The active compounds I are generally included in topical formulations in a concentration of 1—10% based upon the total weight of the formulation, generally 1—6% and, usually, optimally of about 2—4% on the same basis, especially for treatment of the epidermis. For treatment of occular manifestations of the herpes or other virus being treated, concentrations of usually 0.1—1% preferably 0.2—0.5%, are employed. However, in all applications, greater or lesser concentrations may be employed where suitable, e.g., higher concentrations may be employed in the mentioned occular formulations where irritation of the eye does not ensue. The compounds I are topically administered at these concentrations generally 2—8 times per day, preferably 3—4 times per day, as needed. Details of the administration are analogous, in general, to that of such conventional antiviral agents as Vira-A® (adenosine arabinoside; Ara A Parke-Davis).

The compounds I may be employed in human and veterinary medicine for the indications mentioned above in any of the conventional forms for topical application, such as solutions, suspensions lotions, emulsions, creams, ointments, plasters, powders, linaments, salves, aerosols, gels, etc., such that an antiviral effective amount of the compound I is applied. The amount of topical formulation which is administered will depend upon the activity of the particular compound employed and on the severity of the infection and the other fully conventional factors. The amount of active ingredient will be as described above and the amount of topical formulation, e.g. will generally be sufficient to coat the area to be treated.

In general, these topical formulations are non-sprayable, viscous to semi-solid or solid forms comprising a carrier indigenous to topical application and having a dynamic viscosity, preferably greater than that of water. In solid dosage forms, it is often desirable to micronize the compound I to be combined with the various conventional carriers, for example, binders, such as acacia, corn starch or gelatin, disintegrating agents, such as corn starch, guar gum, potato starch or algenic acid, lubricants, such as stearic acid or magnesium stearate, and inert fillers, such as lactose, sucrose or corn starch. Suitable carriers for liquid formulations such as suspensions or solutions, which can optionally be sterile, include oil, water, an alcohol or mixtures thereof, with or without the addition of a pharmaceutically suitable surfactant, suspending agent, or emulsifying agent. These can be used for spreading on the skin or administration to the eye, e.g., as an eye drop. Where suitable, they may contain emolients, perfumes, or moisturizing pigments, etc. to form cosmetically desirable preparations. Such liquid preparations can be formulated suitably with oils, such as fixed oils, such as peanut oil, sesame oil, cottonseed oil, corn oil and olive oil; fatty acids, such as oleic acid, stearic acid and isostearic acid; and fatty acid esters, such as ethyl oleate, isopropyl myristate, fatty acid glycerides and acetylated fatty acid glycerides; with alcohols such as ethanol, isopropanol, hexadecyl alcohol, glycerol and propylene glycol; with glycerol ketals, such as 2,2-dimethyl-1,3-dioxolane-4-methanol; with ethers, such as polyethylene glycol 400; with petroleum hydrocarbons, such as mineral oil and petrolatum; with water; or with mixtures thereof; with or without the addition of a pharmaceutically suitable surfactant, suspending agent or emulsifying agent.

Where appropriate, the topical preparations can also be formulated in the form of soaps and synthetic detergents using conventional carriers for the same. Suitable such soaps include fatty acid alkali metal, ammonium and triethanol amine salts. Suitable detergents include cationic detergents, for example, dimethyl dialkyl ammonium halides, alkyl pyridinium halides, and alkyl amine acetates;

anionic detergents, for example, alkyl, aryl and olefin sulfonates, alkyl, olefin, ether and monoglyceride sulfates, and sulfosuccinates; non-ionic detergents, for example, fatty amine oxides, fatty acid alkanol amides, and polyoxyethylene polyoxypropylene copolymers; and amphoteric detergents, for example, alkyl betaaminopropionates and 2-alkyl imidazoline quaternary ammonium salts; and mixtures thereof. Detergent compositions may be in bar, powder or liquid form and may incorporate foam builders, viscosity control agents, preservatives, emollients, coloring agents, perfumes and solvents.

Aerosol or spray topical preparations may contain a micronized solid or a solution of a compound I and may also contain solvents, buffers, surfactants, perfumes, antioxidants and propellants. They may be applied by means of a propellant under pressure or by means of a compressable plastic spray bottle, a nebulizer or an atomizer without the use of a gaseous propellant.

The topical compositions may also be formulated with other active ingredients such as local anesthetics, such as benzocaine, benzyl alcohol and phenol and their pharmaceutically acceptable salts, as well as antibacterials.

The active compounds I may also be administered by means of a sustained release system whereby they are gradually released at a controlled, uniform rate from an inert or bioerodable carrier by means of diffusion, osmosis, or disintegration of the carrier during the treatment. Such controlled release drug delivery systems may be in the form of patches or bandages applied to the skin or occular inserts placed in the cul de sac of the eye. Such systems permit the treated area to be exposed constantly for prolonged time to a therapeutically effective dosage of a compound I. The unit dosage of the compound administered by means of a sustained release system will approximate the amount of an effective daily dosage multiplied by the maximum number of days during which the carrier is to remain on the body of the host. The sustained release carrier may be in the form of a solid or porous matrix or reservoir and may be formed from one or more natural or synthetic polymers, including modified or unmodified cellulose, starch, gelatin, collagen, rubber, polyolefins, polyamides, polyacrylates, polysiloxanes, and polyimides in mixtures, laminae and copolymers thereof. The compounds I may be incorporated in the sustained release carrier in a pure form or may be dissolved in any suitable liquid or solid vehicle, including the polymer of which the sustained release carrier is formed.

The antiviral activity of the compounds I can be demonstrated in any of several known test systems. For example, *in vivo* antiviral activity may be demonstrated in the nonfatal Herpesvirus paralysis mouse model. The skin at the base of the tail of the mouse (CD—1 strain; males; 18—20 g) is scratched and the scarified site is infected with *Herpesvirus homonis,* type 1 by application with a sterile cotton swab that has been dipped into an innoculum preparation of the same. Ten mice per group are included. A severe hind limb paralysis occurs in untreated mice, usually, at six to eight days after application, day one being considered the day of infection. The mice are treated with the test compound 2, 4 and 6 hours after challenge by topical application with a sterile cotton swab that has been dipped into the test compound preparation. Twenty mice are included in the infected vehicle-treated control group. The animals are observed for 11 days or until paralysis, the end point, is noted. The vehicle is 1.4% polyvinyl alcohol in sterile distilled water. For example, under these test conditions, when applied in topical doses of 3%, 1-(3,4-dichlorophenyl)-5-(morpholin-4-yl)-1-penten-3-one hydrochloride, 5-(4-morpholinyl)-1-(3-(trifluoromethyl)phenyl)-1-penten-3-one hydrochloride and 1-(2,6-dichlorophenyl)-5-(4-morpholinyl)-1-penten-3-one hydrochloride, gave the following percentages of mice paralyzed, respectively: 0%, 40% and 10%.

Significant antiviral activity was also established in this test for, e.g., the first mentioned compound at lower dosages such as 0.25% and above. (In all cases, phosphonoacetate was used as the standard test compound whose activity was also demonstrated in accordance with the regimen.)

The antiviral activity of the compounds I has also been established in the non-fatal hairless mouse lesion test. Hairless mice are innoculated with *Herpesvirus hominis,* type 1 by application via sterile swabs as described above to the lumbar region of the back of the mice, scarified by scratching. The test compounds are applied in optimal doses in a 1.4% solution of polyvinyl alcohol. The mice (male or female) are of the HRS/J strain (18—20 g). During the experiment, they are housed six per cage with food and water ad libitum. In untreated animals, severe ulcerated dermal lesions that follow the nerve pathway to the hind legs will develop by the eighth day after infection. These lesions are scored subjectively as to their degree of severity, i.e., 0 indicating no lesion, to a maximal lesion having a score of 3 (e.g., severe infection, lesions covering one-half or more of the lumbar region). The percent control of lesions is given as 100× the average lesion score for the test group divided by the average lesion score for the control group to which there is administered only the carrier.

Using this procedure, the antiviral activity of 1-(3,4-dichlorophenyl)-5-(morpholin-4-yl)-1-penten-3-one hydrochloride was demonstrated: as doses of 6% and 3%, 87% inhibition of lesions was detected; and at doses Of 1.5% and 0.75%, 81% inhibition of lesions was detected.

Disodium phosphonoacetate was again used as the standard test comopund whose activity was also demonstrated using this regimen (5% in 1.4% polyvinyl alcohol).

The compounds I may be prepared by subjecting the corresponding compound of Formula II to the well-known Mannich reaction as follows:

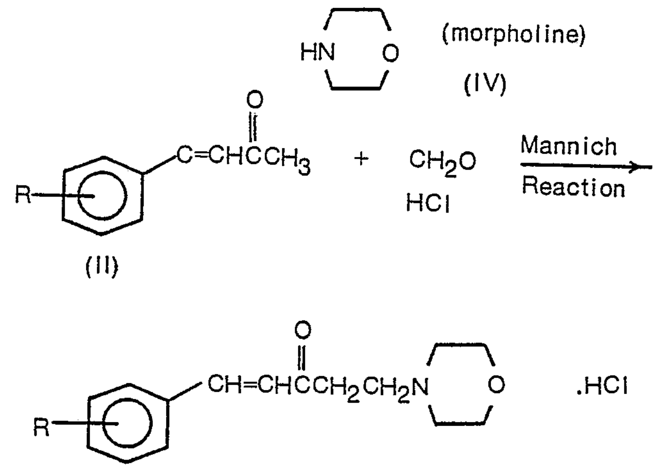


Conditions suitable for conduction of these Mannich reactions are fully conventional, e.g., see *Mannich et al,* Arch. Pharm., *265* 684 (1928).

The Mannich reaction is generally conducted using one equivalent of each of the compounds of Formulae II and IV, along with 1—2 equivalents of formaldehyde. The reaction is usually run in a solvent such as ethanol, acetic acid, 2-propanol or *n*-propanol, preferably ethanol. In a typical run, a mixture of HCl in ethanol (in a weight ratio of generally around 1:5) is first prepared and the amine and formaldehyde are added to it. The reaction mixture is refluxed (generally 70—80°C) for a time of up to 18 hours, typically 16—18 hours. Thereafter, the mixture is cooled and diluted with, e.g., ether or acetone, preferably acetone (about 4—5 volumes based upon the reaction volume). In general, the hydrochloride salt of the compound of Formula I precipitates. The free base can be liberated from the hydrochloride salt by conventional treatment with a strong base, e.g., NaOH. After dilution with water, the compound of Formula I can be extracted with a solvent such as chloroform, ether, ethylacetate, etc. The obtained compound may then be worked-up using conventional methods, such as those described in the examples.

Alternatively, the Mannich reaction may be carried out in acetic acid by adding the compound of Formula II to formaldehyde and the acid addition salt of the compound of Formula IV with HCl, which is the preferred mode for compounds such as dimethylamine which is a gas.

The ketones, of Formula II are all known compounds. They can all be prepared using fully conventional processes such as reaction of methyl lithium with the corresponding compound of Formula III as follows:

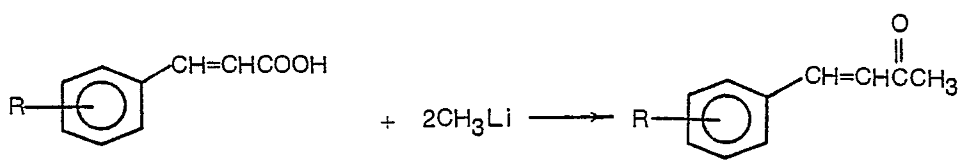


(III) (II)

or by reacting acetone with an aldehyde of Formula V in a conventional aldol condensation using a typical catalyst such as sodium hydroxide, in accordance with the following reaction

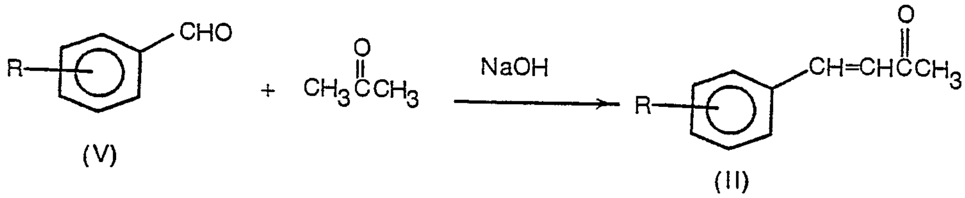


(V) (II)

The former reaction is carried out using two equivalents of methyl lithium for each equivalent of acid of Formula III, one equivalent being required to form the lithium salt and the other to form the product ketone. The reaction is typically carried out in the presence of a solvent such as an ether, i.e., dimethoxyethane, diethylether, dioxane, etc. For each specific case, the reaction is started out in an ice bath in order to determine whether or not the reaction proceeds at such a low temperature. The reaction mixture is then gently warmed in order to accelerate the reaction. in general, suitable

temperatures are 0—40°C, up to the boiling point of the solvent. The reactions are usually conducted at room temperature by adding methyl lithium (e.g., in a commercial 1.8 M concentration in ether) dropwise to a solution of the compound of Formula III in the solvent. Reaction times are generally 2—4 hours. After completion, the reaction medium is poured into 1—2 volumes of a saturated solution of ammonium acetate in order to neutralize the lithium. Two layers are obtained, the product being in the ether layer. After extraction with an aqueous base such as sodium bicarbonate in order to remove the unreacted compound of Formula III, the ether layer is dried over magnesium sulfate and the ether is evaporated. The product of Formula II is then recrystallized or distilled depending on whether the product is a solid or liquid. This reaction is applicable to preparation of most of the intermediates ofFormula II except those wherein R is a reactive group, e.g., alkylsulfonyl. However, for example, the alkyl sulfonyl-containing compound of Formula I may be obtained by oxidizing the corresponding R—S-substituted compound of Formulae II or I, e.g., with two equivalents of metachloroperbenzoic acid at a temperature of 10—60°C for 2—20 hours.

The aldol condensation is carried out under conventional conditions whose details are readily determinable by conventional considerations and/or routine experimentation. The concentration of catalytic base appropriate in a given case will depend upon the nature of the R-group. In general, from 1/2 to 1 volume of a solution of about 50% NaOH is employed per 3 volumes of acetone. In carrying out the reaction, typically, the aldehyde of Formula V, dissolved in from 4—8 volumes of acetone (generally 0.1—0.01 equivalents of aldehyde per equivalent of acetone are employed), is added to the aldol condensation catalyst. A two-phase mixture is obtained. The reaction is run with agitation at a temperature generally below 40°C, preferably 0—40°C, and most preferably 20—30°C, for a time of up to 18 hours in general, usually at least 2—3 hours and preferably 12—16 hours. The product is usually a solid which crystallizes out and is subsequently recrystallized. If not, the reaction medium can be extracted with an organic solvent such as chloroform, diethyl ether, ethyl acetate, methylene chloride, an aromatic hydrocarbon such as benzene, trichlorobenzene, etc. If the product is a liquid, it is vacuum-distilled.

Some of the intermediates of Formula II are commercially available.

The starting materials of Formulae III and V are all conventional compounds and are either commercially available or conventionally preparable by fully conventional procedures such as these disclosed in R. T. Morrison and K. N. Boyd, *Organic Chemistry,* Allyn and Bacon, Boston, Mass. (1976), whose disclosure is incorporated by reference herein.

The addition salts of the compounds of Formula I can be prepared in fully conventional fashion by treating a compound of Formula I with an acid which yields a pharmaceutically acceptable salt. For example, 1—10 equivalents of acid per equivalent of compound of Formula I can be added to the latter at a temperature of −5 to 80°C, typically room temperature, and the reaction conducted for 0.1—5 hours. The reaction is usually conducted in a solvent such as ethylalcohol, water, ether, etc. Suitable acid addition salts include those derived from the following acids: any suitable inorganic or organic acid, e.g., suitable inorganic acids include, for example, hydrochloric, hydrobromic, sulfuric or phosphoric acid, etc.; suitable organic acids include, for example, carboxylic acids, such as acetic, propionic, glycolic, lactic, pyruvic, malonic, succinic, fumaric, malic, tartaric, citric, ascorbic, maleic, hydroxymaleic, benzoic, hydroxybenzoic, phenylacetic, cinnamic, salicylic and 2-phenoxybenzoic, or sulfonic acids such as, for example, methanesulfonic, 2-hydroxyethane sulfonic acid, etc.

The quaternary ammonium salts may be prepared by reacting a compound of Formula I with an alkyl halide at a temperature of 20—70°C. for about 12 to 72 hours in a solvent such as ether, ethyl alcohol, methyl alcohol, methyl ethyl ketone, etc. Other quaternary salts are prepared by standard techniques well known in the art.

In the following examples, all temperatures are set forth uncorrected in degrees Celsius; unless otherwise indicated, all parts and percentages are by weight.

## Example 1

A. *4-(3,4-Dichlorophenyl)-3-buten-2-one:*

10 Grams of 3,4-dichlorobenzaldehyde is added to a mixture of IN sodium hydroxide (120 ml) and acetone (90 ml). The mixture is stirred at room temperature for 3 hours, then diluted with water (90 ml) and extracted with chloroform (2 × 100 ml). the combined chloroform extracts are evaporated to a gummy residue which is then distilled and the b.p. 100°/0.1 mm fraction collected, which fraction solidifies on standing to yield 4-(3,4-dichlorophenyl)-3-buten-2-one; m.p. 53—55°C.

B. *1-(3,4-Dichlorophenyl)-5-morpholin-4-yl-1-penten-3-one hydrochloride:*

To 25.1 g of 4-(3,4-dichlorophenyl)-3-buten-2-one dissolved in 125 ml of ethanol is successively added 5.6 g of paraformaldehyde, 10 g of morpholine and 25 ml of concentrated HCl. The mixture is heated at reflux for 4 hours, cooled to room temperature (about 23°C), diluted with acetone to 1 liter and the acetone dilution maintained at 0°C overnight. The resultant precipitate of 1-(3,4-dichlorophenyl)-5-(morpholin-4-yl)-1-penten-3-one hydrochloride is filtered off and recrystallized from ethanol/methanol (700/200 ml), collected by filtration and vacuum dried for 4 hours at 60°C followed by 18 hours at room temperature; m.p. 199—200°C.

C. *Other Salts of 1-(3,4-dichlorophenyl)-5-morpholin-4-yl-1-penten-3-one:*

The hydrochloride salt prepared in Example 1B above, is converted to the free base in the following manner:

The hydrochloride salt is neutralized with an equivalent amount of 10% aqueous sodium hydroxide and extracted with diethyl ether. The ether extract is then dried and concentrated to afford the free base. 550 mg of citric acid, 428 mg of tartaric acid and 274 mg of methanesulfonic acid are respectively added to individual portions of 2.85 mmoles of the free base. Each solution is heated for 4 hours and is allowed to stand for 1—2 days.

The citrate was solidified with anhydrous ether and recrystallized from isopropyl alcohol, yielding (E)-1-(3,4-dichlorophenyl)-5-(4-morpholinyl)-1-penten-3-one citrate. The tartrate was recrystallized from ethyl alcohol, yielding 1.25 g of (E)-1-(3,4-dichlorophenyl)-5-(4-morpholinyl)-1-penten-3-one tartrate (m.p. 108—110°C). The methanesulfonic acid salt which had solidified was triturated with anhydrous ether and recrystallized from isopropyl alcohol, yielding (E)-1-(3,4-dichlorophenyl)-5-(4-morpholinyl)-1-penten-3-one methanesulfonate hemihydrate (m.p. 105—107°C).

Example 2

A. *4-(3-trifluoromethylphenyl)-3-buten-2-one:* 10.0 g (46.3 mmoles) of m-trifluoromethyl cinnamic acid is dissolved in 300 ml of diethyl ether and the solution chilled to −70°C. 30 ml of a 1.8 molar solution of methyl lithium in ether is added dropwise with stirring. The stirring is maintained at −70°C for 3 hours. The solution is raised to room temperature and stirred overnight. The mixture is then poured into an approximately equal volume of 1 N HCl and two layers obtained. The ether layer is separated and washed with aqueous HCl and then aqueous $NaHCO_3$. The ether extracts are dried ($MgSO_4$) and evaporated. The residue is distilled and the b.p. 90—92°C/0.2 mm fraction of 4-(3-trifluoromethylphenyl)-3-buten-2-one is collected.

B. *5-(4-morpholinyl)-1-(3-trifluoromethylphenyl)-1-penten-3-one hydrochloride:*

A mixture of 5.0 g (23.3 mmoles) of 4-(3-trifluoromethylphenyl)-3-buten-2-one, 0.7 g (23.3 mmoles) of paraformaldehyde, 2.0 g (23.3 mmoles) of morpholine, 30 ml of ethanol and 5 ml of concentrated HCl is heated at reflux for 24 hours. The mixture is cooled and poured into 300 ml of acetone. The acetone solution is chilled overnight at about 0°C. The acetone is evaporated and the residue is recrystallized from ethanol collected by filtration and vacuum dried overnight at 50°C to produce 1.2 g of 5-(4-morpholinyl)-1-(3-trifluoromethylphenyl)-1-penten-3-one hydrochloride. m.p. 192—193°C.

Example 3

A. *4-(2,6-dichlorophenyl)-3-buten-2-one:*

10 g of NaOH is dissolved in 250 ml of water and the solution is added to a solution of 20 g of 2,6-dichlorobenzaldehyde in 200 ml of acetone. The mixture is stirred for one hour at room temperature. Thin layer chromatography is used to determine complete reaction. The mixture is poured into 300 ml of water and the aqueous mixture is extracted with chloroform (2 × 300 ml). The chloroform layers are combined, dried and evaporated. The residue is distilled and the b.p. 102—103°C/0.01 mm fraction is collected to yield 17.6 g of 4-(2,6-dichlorophenyl)-3-buten-2-one.

B. *1-(2,6-dichlorophenyl)-5-(4-morpholinyl)-1-penten-3-one hydrochloride:* 5 g (0.023 mole) of the intermediate prepared in Example 3A is dissolved in 25 ml of ethanol. A mixture of 2 g (0.023 mole) of morpholine, 1.14 g (0.023 mole) of paraformaldehyde and 5 ml of concentrated hydrochloric acid is added thereto. The reaction mixture is refluxed overnight. The mixture is cooled to room temperature and diluted with acetone to a volume of 400 ml. The acetone dilution is maintained at 0°C overnight. The resultant precipitate is collected by filtration and recrystallized from ethyl alcohol. The recrystallized precipitate of 1-(2,6-dichlorophenyl)-5-(4-morpholinyl)-1-penten-3-one hydrochloride is again collected by filtration and vacuum dried. m.p. 193—194°C.

Example 4

A. *4-(4-methylthiophenyl)-3-buten-2-one:*

15.2 g (0.1 mole) of 4-methylthiobenzaldehyde is dissolved in 25 ml of acetone. This solution is diluted with 40 ml of water and 6 ml of water containing 1.3 g of NaOH is added thereto. The mixture is diluted with 300 ml of water and stirred overnight. The resultant mixture is extracted with methylene chloride and the organic layer extracts are evaporated. The residue is recrystallized from toluene giving 8.1 g of 4-(4-methylthiophenyl)-3-buten-2-one. m.p. 99.5—101°C.

B. *(E)-1-[4-methylthio)phenyl]-5-(4-morpholinyl)-1-penten-3-one hydrochloride:*

A mixture of 3.8 g (0.02 mole) of the intermediate product prepared in Example 4A, 600 mg (0.02 mole) of paraformaldehyde, 1.74 g (0.02 mole) of morpholine, 40 ml of ethanol and 4 ml of concentrated HCl is heated at reflux for 18 hours. The resultant solution is diluted with acetone to a volume of 200 ml. The acetone dilution is chilled and maintained at 0°C overnight. The resultant

precipitate is separated by filtration and recrystallized from ethanol. The filtered precipitate is vacuum dried at 50°C to produce 1.7 g of (E)-1-[4-(methylthio)phenyl]-5-(4-morpholinyl)-1-penten-3-one hydrochloride. m.p. 195—196°C.

Example 5

A. *4-(4-methylsulfonylphenyl)-3-buten-2-one:*

3.8 g (20 mmoles) of the product prepared in Example 4A (4-(4-methylthiophenyl)-3-buten-2-one) is dissolved in 100 ml of chloroform and the solution is chilled in an ice bath. There is added dropwise thereto 9.4 g of a 90% solution of m-chloroperbenzoic acid in chloroform (150 ml). After 4 hours at ice bath temperature, the solution is stirred overnight at room temperature. The mixture is extracted with 1N NaOH. The organic layer is dried and evaporated. The residue is recrystallized from hexane yielding 2.6 g of 4-(4-methylsulfonylphenyl)-3-buten-2-one.

B. *5-(4-morpholinyl)-1-(4-methylsulfonylphenyl)-1-penten-3-one hydrochloride:*

6.6 g (30 mmoles) of the intermediate product prepared in Example 5A, 1 g of paraformaldehyde and 27 g (30 mmoles) of morpholine are added to 60 ml of ethanol. There is then added thereto 6 ml of concentrated HCl and the solution is heated at reflux for 18 hours. The reaction solution is then diluted to 200 ml with acetone and maintained at 0°C overnight. The resultant precipitate is separated by filtration and recrystallized from ethyl alcohol/methyl alcohol. The recrystallized product is vacuum dried at 55°C to yield 2.4 g of the trans-isomer of 5-(4-morpholinyl)-1-(4-methylsulfonylphenyl)-1-penten-3-one hydrochloride. m.p. 191—192°C.

Example 6

A. *4-(4-chlorophenyl)-3-buten-2-one:*

To 75 ml of water was added a solution of 28 g of parachlorobenzaldehyde dissolved in 60 ml of acetone. A solution of 2.8 g of NaOH and 12 ml of water, and then a solution of 670 ml of water are added thereto. The reaction mixture is stirred overnight. The resultant precipitate was recrystallized from ethanol yielding 32 g of 4-(4-chlorophenyl)-3-buten-2-one. m.p. 47—50°C.

B. *5-(4-morpholinyl)-1-(4-chlorophenyl)-1-penten-3-one hydrochloride:*

6 g (30 mmoles) of the intermediate product prepared in Example 6A 1g (a 10% excess) of paraformaldehyde, 2.6 g (30 mmoles) of morpholine, 60 ml of ethanol and 6 ml of concentrated hydrochloric acid are mixed and the mixture is heated for 18 hours at reflux. The reaction mixture is then diluted to 200 ml with acetone and is maintained at 0°C overnight. The resultant precipitate is separated by filtration and is recrystallized from ethyl alcohol, yielding 3.1 g of 5-(4-morpholinyl)-1-(4-chlorophenyl)-1-penten-3-one hydrochloride. m.p. 204—205°C.

Example 7

A. *4-(4-methoxyphenyl-3-buten-2-one:*

A solution of 28 g of p-methoxybenzaldehyde, dissolved in 70 ml of acetone, is added to an equal volume of water and the reaction mixture is stirred vigorously. To the stirring mixture is added a solution of 2.8 g of sodium hydroxide dissolved in 15 ml of water, followed by an additional 600 ml of water. The resultant precipitate is collected by filtration, and dried and recrystallized from the minimum quantity of hexane to yield 4-(4-methoxyphenyl)-3-buten-2-one; m.p. 72—73°C.

B. *(E)-1-(4-methoxyphenyl)-5-(4-morpholinyl)-1-penten-3-one hydrochloride:*

5.28 g (0.03 mole) of the product of Example 7 A, 2.6 g (0.03 mole) of morpholine and 2 g (0.066 mole) of paraformaldehyde are added to 60 ml of ethanol. 6 ml of concentrated HCl is added to the solution and the mixture is heated at reflux temperature for 18 hours. The resultant mixture is diluted with acetone to 200 ml and chilled. The resultant solution is evaporated to a small volume and diluted with ethanol (100 ml). On standing at room temperature, crystals form. The precipitate is filtered off and recrystallized from ethanol/methanol, yielding (E)-1-(4-methoxyphenyl)-5-(4-morpholinyl)-1-penten-3-one hydrochloride; m.p. 180—181°C.

Example 8

The compounds I can be administered topically to a patient as an antiviral agent, especially in the treatment of various manifestations of herpes virus.

As the experimental results summarized above established, the compounds I display antiviral effects in mice under conditions comparable to those under which administration of known antiviral agents such as phosphonoacetate achieves antiviral effects. Similar results can be established for other known antiviral agents such as vira-A (Parke Davis) or Stoxil (SKF). Thus, the compounds I will be administered to humans, e.g., for the same indications as those seen in mice for such known compounds.

Of course, the dosage amounts for the compounds I will be adjusted for their particular potencies as compared with those of such known agents as determined, for example, via the protocols discussed

above or other conventional protocols.

For example, the compounds I can be administered topically to a 70 kg adult 2—8 times per day in conventional topical formulations wherein it is present in a concentration of 1—10% based upon the total weight of the formulation, generally about 2—4%, e.g., for application to the epidermis for alleviation of symptoms of a herpes virus.

Example 9

Solution

| | |
|---|---|
| 1-(3,4-dichlorophenyl)-5-(morpholin-4-yl)-1-penten-3-one hydrochloride | 0.85 g |
| Alcohol | 78.9 ml |
| Isopropyl Myristate | 5.0 g |
| Polyethylene Glycol 400 (Av. M.W. 400) | 10.0 g |
| Purified Water sufficient to make | 100 ml |

Combine the alcohol, isopropyl myristate and polyethylene glycol 400 and dissolve the drug substance therein. Add sufficient purified water to give 100 ml.

Example 10

Powder

| | |
|---|---|
| 1-(3,4-dichlorophenyl)-5-(morpholin-4-yl)-1-penten-3-one hydrochloride | 1% w/w |
| Silicon dioxide, anhydrous | 0.5% w/w |
| Corn starch, lactose, fine powder—each, with the total sufficient to make | 50 kg |

Example 11

Hand Lotion

| | |
|---|---|
| 5-(4-morpholinyl)-1-phenyl-1-penten-3-one hydrochloride (not within the scope of this invention) | 0.15 g |
| Isostearic acid | 10.0 g |
| Stearic acid | 8.0 g |
| Poloxamer 235 | 5.0 g |
| Propylene glycol | 10.0 g |
| Deionized water sufficient to make | 100.0 ml |

Example 12

Liquid Soap

| | |
|---|---|
| 5-(4-morpholinyl)-1-phenyl-1-penten-3-one hydrochloride (not within the scope of this invention) | 0.3 g |
| Green soap tincture, NF | 100 ml |

Example 13

Liquid Detergent

| | |
|---|---|
| 1-(3,4-dichlorophenyl)-5-(morpholin-4-yl)-1-penten-3-one hydrochloride | 0.025 g |
| Miranol SM Concentrate ® (Miranol Chem. Co., Irvington, N.J.) (35% 1-Carboxymethyl-3,4-dihydro-1-(2-hydroxyethyl)-2-nonyl-1H-imidazolium hydroxide, disodium salt, 5% NaCl, pH 8.9—9.1) | 25.0 g |
| Laureth-4 (Monolauryl ethers of polyoxyethylene glycols containing an average of 4 oxyethylene groups) | 2.0 g |
| Deionized water sufficient to make | 100 ml |

The preceding examples can be repeated with similar success by substituting the general or specifically described reactants and/or operating conditions of this invention for those used in the preceding examples.

**Claims for the Contracting States BE CH DE FR GB IT LI NL SE**

1. A pharmaceutical composition with antiviral activity for administration topically, comprising a pharmaceutically acceptable carrier for topical compositions and an antivirally effective amount of a 5-(4-morpholinyl)-1-(substituted phenyl)-1-penten-3-one compound of the formula I

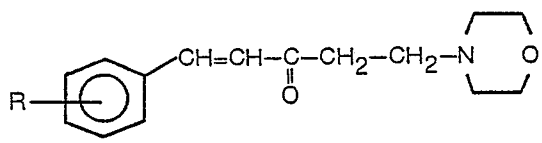

(I)

wherein R is either (a) a mono substituted moiety selected from methoxy, 4-halo, $CF_3$, —S—$C_{1-4}$-alkyl, —$SO_2C_{1-4}$-alkyl, or (b) a dihalo moiety; or a pharmaceutically acceptable salt thereof with an acid or a quaternary ammonium salt thereof.

2. The pharmaceutical composition of claim 1 wherein the compound of Formula I is 5-(4-morpholinyl)-1-(3,4-dichlorophenyl)-1-penten-3-one or the hydrochloride salt thereof.

3. The pharmaceutical composition of claim 1 which is a lotion, cream, ointment, linament, salve, soap or detergent having a dynamic viscosity greater than that of water or an aerosol spray.

4. The pharmaceutical composition of claim 3 wherein the antiviral compound is 5-(4-morpholinyl)-1-(3,4-dichlorophenyl)-1-penten-3-one or the hydrochloride salts thereof.

5. Use of the 5-(4-morpholinyl)-1-(substituted phenyl)-1-penten-3-one compounds I according to claims 1 to 4 for the preparation of a pharmaceutical composition with antiviral activity for topical administration.

6. A quaternary salt of the 5-(4-morpholinyl)-1-(substituted phenyl)-1-penten-3-one compound of the general formula (I) from claim 1.

7. A process for preparing a quaternary ammonium salt of 5-(4-morpholinyl)-1-(substituted phenyl)-1-penten-3-one of the general formula I according to claim 1, which comprises reacting a compound of the general formula II

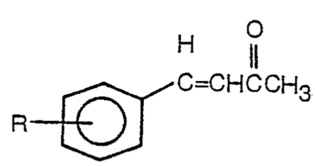

(II)

wherein R is as defined above;
with morpholine in the presence of formaldehyde and HCl; optionally reacting a resultant hydrochloride salt with a base to release the corresponding free base; and reacting the free base with a $C_{1-4}$ alkyl halide to form the corresponding quaternary ammonium salt.

**Claims for the Contracting State AT**

1. Process for preparing a pharmaceutical composition with antiviral activity for topical administration, comprising combining a pharmaceutically acceptable carrier for topical compositions with an antivirally effective amount of a 5-(4-morpholinyl)-1-(substituted phenyl)-1-penten-3-one compound of the general formula I

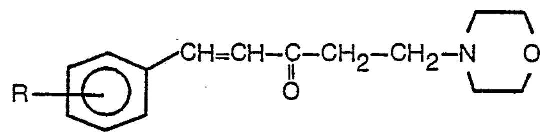

(I)

wherein R is either (a) a mono substituted moiety selected from methoxy, 4-halo, 3-halo, $CF_3$, —S—$C_{1-4}$-alkyl, —$SO_2C_{1-4}$-alkyl, or (b) a dihalo moiety; or a pharmaceutically acceptable salt thereof with an acid or a quaternary ammonium salt thereof.

2. Process according to claim 1 wherein the compound of Formula I is 5-(4-morpholinyl)-1-(3,4-dichlorophenyl)-1-penten-3-one or the hydrochloride salt thereof.

3. A process for preparing a quaternary ammonium salt of a 5-(4-morpholinyl)-1-(substituted phenyl)-1-penten-3-one compound of the general formula I according to claim 1,which comprises reacting a compound of the general formula II

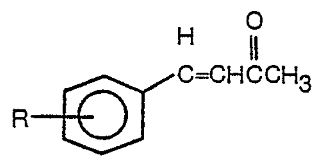

(II)

wherein R is as defined in claim 1, with morpholine in the presence of formaldehyde and HCl, optionally reacting a resultant hydrochloride salt with a base to release the corresponding free base and reacting the free base with a $C_{1-4}$-alkyl halide to form the corresponding quaternary ammonium salt.

**Revendications pour les Etats contractants BE CH LI DE FR GB IT NL SE**

1. Une composition pharmaceutique à activité antivirale pour l'administration topique, comprenant un véhicule acceptable pour l'usage pharmaceutique pour compositions topiques et une quantité antivirale efficace d'un dérivé de 5-(4-morpholinyl)-1-(phényle substitué)-1-pentène-3-one de formule I

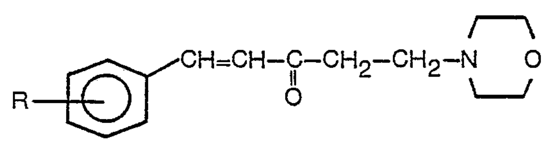

(I)

dans laquelle R représente soit (a) un motif mono-substitué choisi parmi les motifs méthoxy, 4-halogéno, 3-halogéno, $CF_3$, —S-alkyle en $C_1$—$C_4$, —$SO_2$-alkyle en $C_1$—$C_4$ soit (b) un motif dihalogéné; ou un sel acceptable pour l'usage pharmaceutique d'un tel composé avec un acide ou un sel d'ammonium quaternaire d'un tel composé.

2. La composition pharmaceutique selon la revendication 1, dans laquelle le composé de formule I est la 5-(4-morpholinyl)-1-(3,4-dichlorophényl)-1-pentène-3-one ou son chlorhydrate.

3. La composition pharmaceutique de la revendication 1, qui est une lotion, une crème, une pommade, un liniment, un onguent, un savon, un détergent ayant une viscosité dynamique supérieure à celle de l'eau, ou un aérosol.

4. La composition pharmaceutique de la revendication 3, dans laquelle le composé antiviral est la 5-(4-morpholinyl)-1-(3,4-dichlorophényl)-1-pentène-3-one ou son chlorhydrate.

5. L'utilisation des dérivés de 5-(4-morpholinyl)-1-(phényle substitué)-1-pentène-3-one de formule I selon les revendications 1 à 4, pour la préparation d'une composition pharmaceutique à activité antivirale pour l'administration topique.

6. Un sel quaternaire du dérivé de 5-(4-morpholinyl)-1-(phényle substitué)-1-pentène-3-one de formule générale I de la revendication 1.

7. Un procédé pour préparer un sel d'ammonium quaternaire de la 5-(4-morpholinyl)-1-(phényle substitué)-1-pentène-3-one de formule générale I selon la revendication 1, qui consiste à faire réagir un composé de formule générale II:

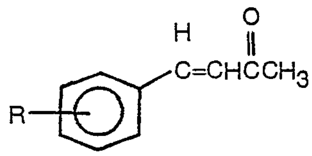

(II)

dans laquelle R a les significations indiquées ci-dessus, avec la morpholine, en présence de formaldéhyde et d'HCl; puis à faire réagir facultativement un chlorohydrate obtenu avec une base pour libérer la base libre correspondante; et à faire réagir la base libre avec un halogénure d'alkyle en $C_1$—$C_4$ pour former le sel d'ammonium quaternaire correspondant.

**Revendications pour l'Etat contractant: AT**

1. Procédé de préparation d'une composition pharmaceutique à activité antivirale pour l'administration topique qui consiste à combiner un véhicule acceptable pour l'usage pharmaceutique pour compositions topiques avec une quantité antivirale efficace d'un dérivé de 5-(4-morpholinyl)-1-(phényle substitué)-1-pentène-3-one de formule générale I

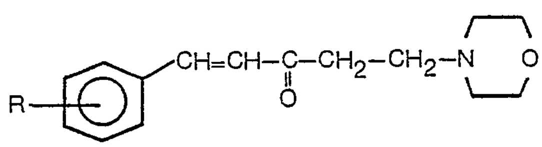

(I)

dans laquelle R représente soit (a) un motif monosubstitué choisi parmi les motifs méthoxy, 4-halogéno, 3-halogéno, $CF_3$, —S-alkyle en $C_1$—$C_4$, —SO-alkyle en $C_1$—$C_4$ soit (b) un motif dihalogéné; ou un sel acceptable pour l'usage pharmaceutique d'un tel composé avec un acide ou un sel d'ammonium quaternaire d'un tel composé.

2. Procédé selon la revendication 1, dans laquelle le composé de formule I est la 5-(4-morpholinyl)-1-(3,4-dichlorophényl)-1-pentène-3-one ou son chlorhydrate.

3. Un procédé pour préparer un sel d'ammonium quaternaire d'un dérivé de 5-(4-morpholinyl)-1-(phényle substitué)-1- pentène-3-one de formule générale I selon la revendication 1, qui consiste à faire réagir un composé de formule générale II

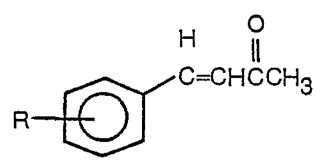

(II)

dans laquelle R a été défini dans la revendication 1, avec la morpholine en présence de formaldéhyde et d'HCl, et à faire réagir facultativement un chlorhydrate obtenu avec une base pour libérer la base libre correspondante et à faire réagir la base libre avec un halogénure d'alkyle en $C_1$—$C_4$ pour former le sel d'ammonium quaternaire correspondant.

**Patentansprüche für die Vertragsstaaten BE CH LI DE FR GB IT NL SE**

1. Arzneimittel mit antiviraler Wirkung zur lokalen Anwendung, enthaltend einen pharmazeutisch verträglichen Träger für lokal anwendbare Arzneimittel und eine antiviral wirksame Menge einer 5-(4-Morpholinyl)-1-(substituiertesphenyl)-1-penten-3-on-Verbindung der Formel I

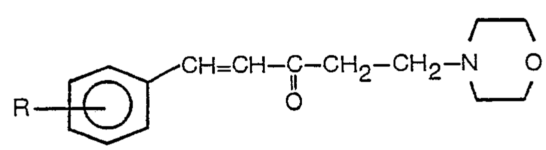

(I)

in der R entweder
(a) ein Monosubstituent aus der Gruppe Methoxy, 4-Halogen, 3-Halogen, $CF_3$, —S—$C_{1-4}$-Alkyl, —$SO_2C_{1-4}$-Alkyl oder
(b) ein Dihalogen-Substituent ist, oder ein pharmazeutisch verträgliches Salz mit einer Säure oder ein quartäres Ammoniumsalz.

2. Arzneimittel nach Anspruch 1, wobei die Verbindung der Formel I das 5-(4-Morpholinyl)-1-(3,4-dichlorophenyl)-1-penten-3-on oder dessen Hydrochlorid ist.

3. Arzneimittel nach Anspruch 1 als Lotion, Creme, Salbe, Einreibemittel, Seife oder Waschmittel

mit einer dynamischen Viskosität, die größer ist als die von Wasser oder einem Aerosolspray.

4. Arzneimittel nach Anspruch 3, wobei die antivirale Verbindung das 5-(4-Morpholinyl)-1-(3,4-dichlorophenyl)-1-penten-3-on oder dessen Hydrochlorid ist.

5. Verwendung der 5-(4-Morpholinyl)-1-(substituiertes phenyl)-1-penten-3-on-Verbindungen der Formel I gemäß Anspruch 1 bis 4 zur Herstellung von Arzneimitteln mit antiviraler Wirkung zur lokalen Anwendung.

6. Quartäres Salz einer 5-(4-Morpholinyl)-1-(substituiertes-phenyl)-1-penten-3-on-Verbindung der Formel I gemäß Anspruch 1.

7. Verfahren zur Herstellung von quartären Ammoniumsalzen von 5-(4-Morpholinyl)-1-(substituiertes-phenyl)-1-penten-3-on-Verbindungen der Formel I nach Anspruch 1, dadurch gekennzeichnet, daß man eine Verbindung der Allgemeinen Formel II

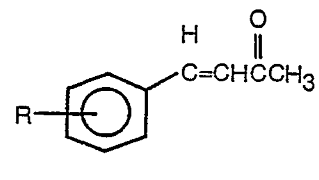

(II)

in der R die vorstehend angegebene Bedeutung, hat, mit Morpholin in Gegenwart von Formaldehyd und HCl umsetzt, gegebenenfalls ein erhaltenes Hydrochloridsalz mit einer Base umsetzt und die freie Base in Freiheit setzt, und die freie Base mit einem $C_{1-4}$-Alkylhalogenid unter Bildung des entsprechenden quartären Ammoniumsalzes umsetzt.

**Patentansprüche für den Vertragsstaat: AT**

1. Verfahren zur Herstellung eines Arzneimittels mit antiviraler Aktivität zur lokalen Anwendung, dadurch gekennzeichnet, daß man einen pharmazeutisch verträglichen Träger für lokal anwendbare Mittel kombiniert mit einer antiviral wirksamen Menge einer 5-(4-Morpholinyl)-1-substituierten-phenyl)-1-penten-3-on-Verbindung der Formel I

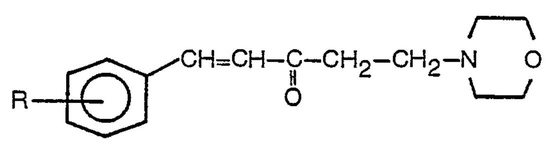

(I)

in der R entweder

a) Methoxy-, 4-Halogen, 3-Halogen, $CF_3$, —S—$C_{1-4}$-Alkyl, oder —$SO_2$—$C_{1-4}$-Alkyl-Monosubstituent oder

b) ein Dihalogen-Substituent ist, oder mit einem Salz einer pharmazeutisch verträglichen Säure oder mit einem quatären Ammoniumsalz.

2. Verfahren nach Anspruch 1, dadurch gekennzeichnet, daß man als Verbindung der Formel I 5-(4-Morpholinyl)-1-(3,4-dichlorophenyl)-1-penten-3-on oder dessen Hydrochlorid verwendet.

3. Verfahren zur Herstellung eines quartären Ammoniumsalzes einer 5-(4-Morpholinyl)-1-(substituierten-phenyl)-1-penten-3-on -Verbindung der Formel I nach Anspruch 1, dadurch gekennzeichnet, daß man eine Verbindung der allgemeinen Formel II

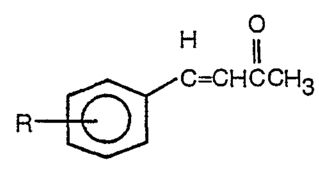

(II)

in der R die in Anspruch 1 angegebene Bedeutung hat, mit Morpholin in Gegenwart von Formaldehyd und HCl umsetzt, gegebenenfalls aus dem erhaltenen Hydrochlorid mit einer Base die entsprechende freie Base in Freiheit setzt, und die freie Base mit einem $C_{1-4}$-Alkylhalogenid zur Bildung des entsprechenden quartären Ammoniumsalzes umsetzt.